# FASCICULE DE BREVET EUROPEEN

(11) **EP 0 709 062 B1**
(45) Date de publication et mention de la délivrance du brevet: **30.07.2003**
(21) Numéro de dépôt: 95420300.6
(22) Date de dépôt: 27.10.1995
(51) Int. Cl.: A61B 17/15

(54) **Instrumentation de coupe tibiale**
Tibiaresektionsinstrument
Tibial cutting instrument

(30) Priorité: 27.10.1994 FR 9413094
(43) Date de publication de la demande: 01.05.1996
(73) Titulaire: Merck Biomaterial France, 26000 Valence (FR); Bascoulergue, Gérard, F-62520 Le Touquet (FR); Charret, Philippe, 69270 Fontaine sur Saone (FR); Fayard, Jean-Philippe, F-42170 St. Just St. Rambert (FR); Hulin, Paul Henri, 89000 Auxerre (FR); Peyrot, Jacques, 69160 Tassin la Demi Lune (FR)
(72) Inventeur: Bascoulergue, Gérard, F-62520 Le Touquet (FR); Charret, Philippe, F-69270 Fontaine sur Saone (FR); Dupre-Latour, Laurent, F-42580 L'Etrat (FR); Fayard, Jean-Philippe, F-42170 St Just St Rambert (FR); Hulin, Paul H., F-89000 Auxerre (FR); Peyrot, Jacques, F-69160 Tassin la Demi Lune (FR); Collomb, Jean, F-26800 Portes les Valence (FR)
(74) Mandataire: Ropital-Bonvarlet, Claude

(56) Documents cités:
- EP-A- 0 538 152
- WO-A-88/07840
- US-A- 4 759 350

## Description

La présente invention concerne le domaine de la chirurgie orthopédique et elle vise, plus particulièrement, le matériel, mis en oeuvre à titre d'aide opératoire, pour la réalisation d'interventions chirurgicales visant à adapter un système articulaire artificiel propre à remplacer une articulation naturelle défaillante.

L'objet de l'invention concerne une instrumentation ancillaire propre à la chirurgie orthopédique du genou et, plus spécifiquement, à l'implantation de prothèses à caractère pluri ou unicompartimental.

Dans le domaine technique général ci-dessus, il est connu depuis longtemps déjà de proposer des systèmes articulaires artificiels visant à remplacer l'articulation naturelle, constituée par la conformation épiphysaire basse ou distale du fémur, par la conformation épiphysaire complémentaire haute ou proximale du tibia et par l'élément fémoro-patellaire.

De telles propositions s'appuient sur une constitution d'un système articulaire artificiel, à partir de deux éléments principaux destinés à être respectivement adaptés, après résection osseuse, sur la conformation épiphysaire basse du fémur et sur la conformation épiphysaire complémentaire haute du tibia.

L'objet de l'invention concerne, plus particulièrement, le matériel ancillaire ou d'aide opératoire, permettant de réaliser, de façon précise, une résection osseuse de la ou des conformations épiphysaires hautes du tibia, en vue de l'implantation, de la mise en place ou de l'insertion d'une prothèse uni ou pluricompartimentale.

Dans le domaine technique ci-dessus, une étape opératoire importante réside dans la préparation du plateau tibial, en vue de réaliser une résection de la masse osseuse juste suffisante en fonction de l'objectif à atteindre qui est de réparer ou corriger, soit une usure prématurée de la surface articulaire concernée, soit une conformation anatomique originelle, responsable d'une angulation inappropriée des systèmes articulaires et favorisant une usure ou un dysfonctionnement prématuré.

On conçoit qu'il est absolument nécessaire de pouvoir pratiquer, sur le massif osseux concerné, une résection osseuse suivant un ou des plans qui correspondent à un appui nécessaire pour l'insertion ou l'implantation d'une prothèse de remplacement.

Le calcul de l'orientation du plan par rapport à un ou plusieurs axes anatomiques ou fonctionnels de l'os concerné, revêt une importance capitale pour la bonne implantation, la bonne tolérance ultérieure et le bon fonctionnement de la prothèse implantée.

Des instrumentations ancillaires tibiales, pour la résection osseuse de tout ou partie du plateau tibial, ont, à cet effet, été proposées par la technique antérieure.

Il s'avère, toutefois, que les instrumentations ancillaires proposées à cette fin ne permettent pas de prendre en compte l'ensemble des exigences d'orientation et de réglage respectant l'axe anatomique du tibia dans le plan sagittal, la détermination de cet axe dans le plan frontal, l'orientation du ou des massifs condyliens naturels devant être réséqués, ainsi que la profondeur de résection de la masse osseuse.

Par ailleurs, il peut être considéré que les instrumentations ancillaires tibiales, actuellement connues, s'appuient généralement sur un moyen de visée correspondant, soit à un axe intramédullaire, soit à un axe extramédullaire, soit à la combinaison des deux.

Ainsi, la demande de brevet EP 0 538 152 décrit une instrumentation ancillaire tibiale comprenant une tige de visée extramédullaire télescopique portant, à l'une de ses deux extrémités, une potence de support d'une tige de visée intramédullaire et, à l'autre extrémité, une fourche d'écartement fine adaptable sur une partie basse du tibia. Cette instrumentation comprend, enfin, un guide de coupe adaptable sur la partie de la tige télescopique voisine de la potence et un palpeur amovible de réglage du guide de coupe.

La mise en place de cette instrumentation ancillaire est assurée par une introduction de la tige de visée intramédullaire, sensée suivre l'axe du canal médullaire, et par un engagement de la fourche sur le tibia.

Or, d'une part, la détermination de l'axe du canal médullaire est particulièrement délicate et, d'autre part, le perçage profond, effectué pour le passage de la tige de visée intramédullaire, interdit toute possibilité de correction. Enfin, la mise en place de la fourche sur la partie basse de la jambe est fonction de l'anatomie de ladite jambe, de sorte que la visée extramédullaire possède un fort caractère aléatoire.

Il apparaît donc qu'une telle instrumentation n'est pas à même de fournir un référentiel précis, à partir duquel les différentes angulations ou orientations, devant être respectées ou recherchées, peuvent être précisément définies. Il est ainsi fréquent que les plans de résection choisis donnent lieu à des implantations de prothèses ou de systèmes articulaires induisant des dysfonctionnements responsables, notamment, de fatigue ligamentaire ou de désolidarisation intempestive entre la prothèse artificielle et la masse osseuse naturelle.

L'objet de l'invention est de surmonter les inconvénients actuellement connus et pouvant être portés au compte des instrumentations ancillaires tibiales proposées et il vise, à cet effet, une nouvelle instrumentation ancillaire, simple de conception et d'utilisation et dont les moyens techniques permettent de déterminer avec précision l'orientation, l'alignement et la position des moyens d'aide à l'implantation permettant de réaliser des résections osseuses selon des plans relatifs favorables à l'implantation de prothèses à même de fonctionner dans des conditions de compatibilité certaine avec la ou les contreparties fémorales adaptées.

L'objet de l'invention vise une instrumentation ancillaire tibiale qui est à même de favoriser le travail préparatoire du chirurgien, en lui fournissant des axes et plans de référence et d'appui, à position, orientation ou sens éventuellement réglables, et qui est à même de lui fournir un domaine référentiel précis, à partir duquel une implantation de prothèse tibiale, unie ou pluricompartimentale, peut être réalisée avec le maximum de sécurité.

Pour atteindre les objectifs ci-dessus, l'instrumentation ancillaire tibiale conforme à l'invention comprend :
- une potence de pointage sur le plateau tibial adaptée, de façon amovible, sur la partie supérieure ou proximale de la tige de visées et pourvoue d'au moins une broche de pointage sur le plateau tibial
- un étrier d'alignement malléolaire délimitant une coulisse d'orientation,
- une noix de réglage et d'immobilisation liant la partie terminale inférieure ou distale de la tige de visée à la coulisse,
- un support de tête réglable, porté par la partie de la tige voisine de la potence,
- et une tête de travail amovible et réglable sur le support de tête et apte à recevoir un palpeur de réglage et un guide de coupe.

Diverses autres caractéristiques ressortent de la description faite ci-dessous en référence aux dessins annexés qui montrent, à titre d'exemple non limitatif, une forme de réalisation de l'objet de l'invention.

La **fig. 1** est une élévation prise parallèlement au plan sagittal et montrant, partie en coupe, l'instrumentation ancillaire tibiale conforme à l'invention.

La **fig. 2** est une vue de face prise selon la ligne **II-II** de la **fig. 1.**

La **fig. 3** est une coupe-élévation partielle, en partie arrachée, montrant, à plus grande échelle, un détail de réalisation de l'objet de l'invention.

La **fig. 4** est une vue en plan, partie en coupe, prise à plus grande échelle, sensiblement selon la ligne **IV-IV** de la **fig. 1.**

La **fig. 5** est une coupe prise, à plus grande échelle, selon la ligne **V-V** de la **fig. 2.**

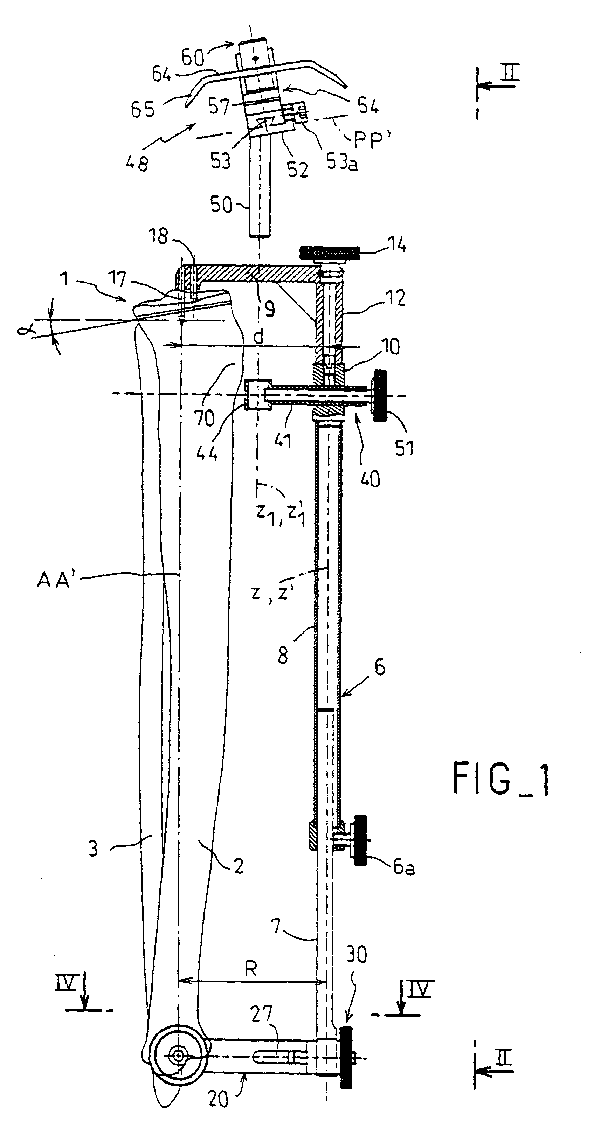

Les **fig. 1** à **3** montrent l'instrumentation ancillaire tibiale, conforme à l'invention, prévue pour permettre d'assurer, dans des conditions optimales, la résection osseuse de tout ou partie d'un plateau tibial **1**, lorsqu'il convient d'adapter, sur ce plateau, une prothèse tibiale unie ou pluricompartimentale, à même d'offrir une complémentarité de surface pour la coopération avec une prothèse complémentaire adaptée sur le ou les condyles épiphysaires bas d'un fémur.

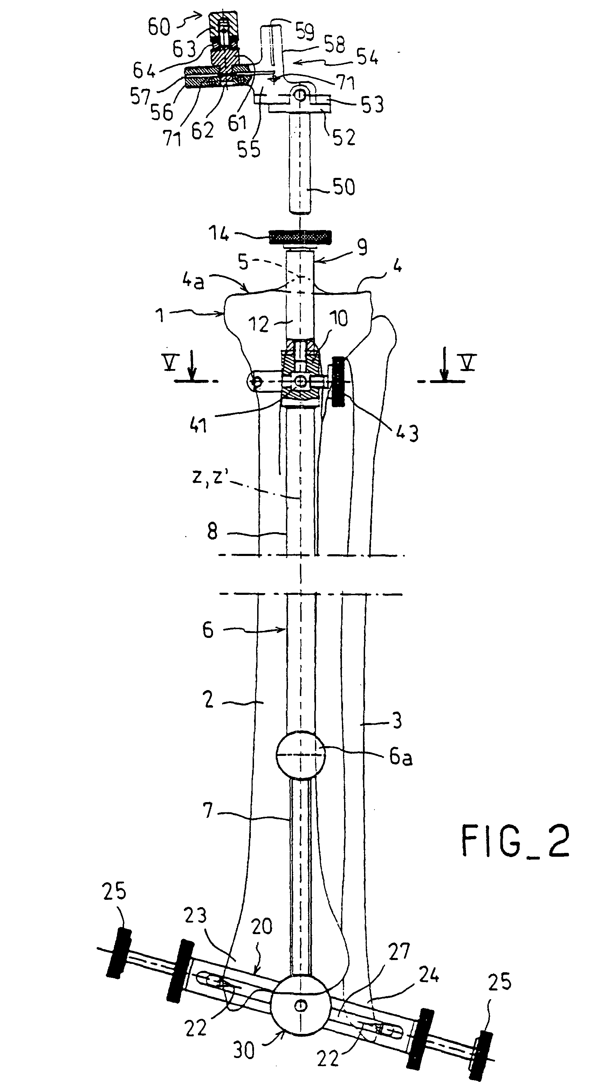

A titre d'exemple, les **fig. 1** et **2** montrent une application à un tibia **2** correspondant à une jambe gauche, un tel tibia **2,** associé à son péroné **3,** étant vu de face sur la **fig. 2.**

Pour réaliser la résection totale ou partielle du plateau tibial **1,** il convient de prendre en compte la caractéristique anatomique de ce dernier qui fait intervenir l'existence de deux glènes **4** latérales, séparées par un massif épineux **5.** Une résection de l'une ou des deux glènes doit prendre en compte, obligatoirement, leur plan d'alignement naturel qui peut faire intervenir généralement, tel que cela ressort de la **fig. 1,** une déclivité postérieure α.

L'objet de l'invention est de proposer une instrumentation ancillaire tibiale qui permette de prendre en compte les différentes caractéristiques anatomiques ou morphologiques, ainsi que les exigences de résection en fonction des dommages de l'articulation naturelle ou, encore, des angulations naturelles anatomiques, que présentent les segments articulaires intéressés par l'articulation visée.

L'instrumentation ancillaire tibiale comprend, tout d'abord, une tige télescopique réglable de visée extramédullaire **6** qui comporte une partie distale **7** et une partie proximale **8.** Les parties **7** et **8** coulissent relativement et sont susceptibles d'être réglées en longueur par l'intermédiaire d'une vis de réglage ou analogue **6a.**

A titre d'exemple, la partie distale **7** est constituée par une barre ou analogue, alors que la partie proximale **8** est formée par un segment de tube à l'intérieur duquel la barre **7** peut coulisser en étant, de préférence, guidée.

L'association de la barre **7** et du tube **8** définit un axe de visée extramédullaire **z-z'**, par référence à une orientation généralement verticale qui lui est conférée par analogie avec l'axe vertical mécanique **A-A'** du tibia **2.**

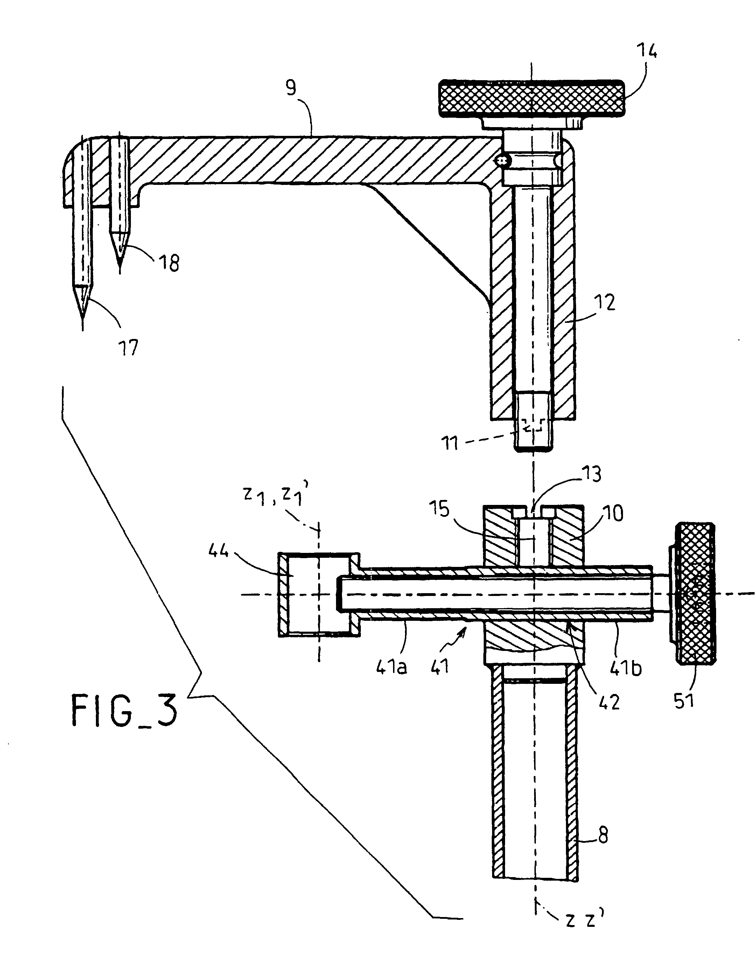

La tige de visée extramédullaire **6** est pourvue, en partie proximale ou supérieure, d'une potence de pointage **9** qui est adaptée, de façon amovible, sur un manchon **10** du tube **8,** par un emmanchement ou une liaison d'orientation angulaire obligée, telle que celle fournie par un ergot **11** de la base du fût **12** de la potence et par un cran **13** présenté par le manchon **10 (fig. 3).** Le fût **12** porte une vis axiale **14** à même de coopérer avec un taraudage **15** présenté axialement par le manchon **10.** Le fût **12** peut ainsi, par ce moyen, être monté en bout du tube **8** pour être aligné avec l'axe **z-z'**, tout en étant immobilisé angulairement selon une orientation prédéterminée. La potence **9** porte en bout une broche principale de pointage **17,** s'étendant parallèlement et à une distance **d** de l'axe du fût **12.** La broche de pointage **17** est, avantageusement, associée à une broche parallèle **18,** dite d'immobilisation, de longueur plus courte que la broche **17.**

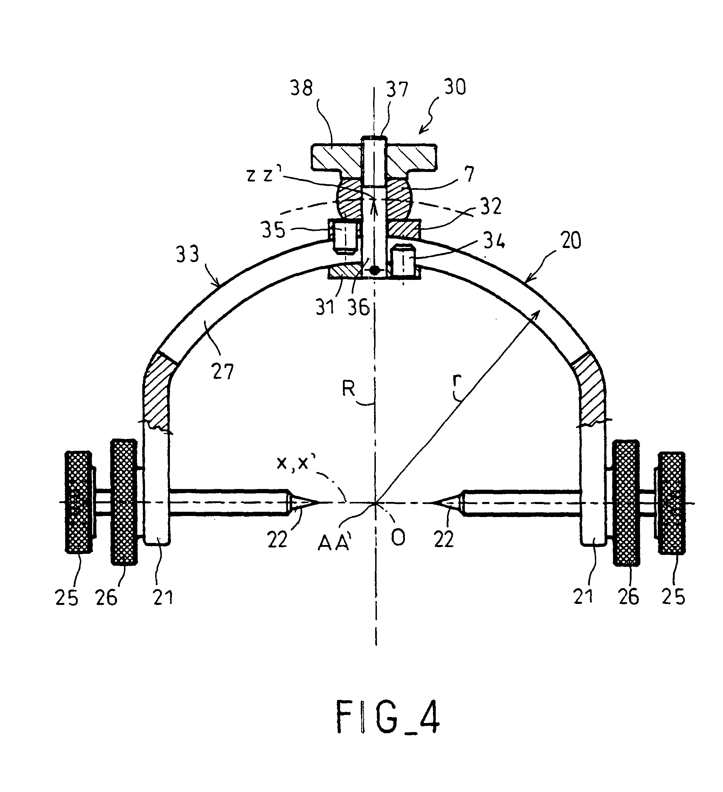

L'instrumentation ancillaire tibiale comprend, par ailleurs, un étrier **20** d'alignement malléolaire qui présente, tel que cela apparaît plus particulièrement à la **fig. 4,** deux branches **21,** de préférence mais non exclusivement, parallèles, portant deux contre-pointes opposées **22** destinées à être engagées, par pénétration ponctuelle perforante, au moins dans la couche corticale des malléoles **23** et **24** appartenant, respectivement, à la conformation épiphysaire basse ou distale du fémur **2** et à celle du péroné associé **3.** Les contre-pointes **22** sont montées sur ou dans les branches **21** par l'intermédiaire de vis de serrage **25,** de préférence micrométriques, permettant de régler leur rapprochement ou écartement relatif, et par l'intermédiaire d'organes de serrage **26,** tels que des contre-écrous de blocage. Les contre-pointes **22** définissent un axe d'alignement malléolaire **x-x'** dont la vocation est de pouvoir passer par l'axe **A-A',** tel que cela ressort de l'explication qui suit.

L'étrier d'alignement malléolaire **20** délimite, dans la partie comprise entre les branches **21,** une coulisse **27,** dite d'orientation, qui présente la particularité d'être définie par une portion de cercle déterminé par un rayon **r** centré sur un centre **O** situé sur l'axe **x-x'** et, par construction, à égale distance des branches **21** parallèles.

La coulisse d'orientation **27** est destinée à coopérer avec une noix **30,** dite de réglage, d'immobilisation et d'orientation, prévue pour lier l'étrier **20** à la partie terminale inférieure ou distale de la tige de visée **6** et, plus particulièrement, la barre **7.** La noix **30** comprend deux demi-noix **31** et **32** qui sont montées pour coopérer avec la partie de l'étrier **20,** telle que l'âme **33,** liant les deux branches **21** et dans laquelle la coulisse **27** est ménagée. Les moyens de coopération peuvent être constitués, à cette fin, par des doigts **34** appartenant, respectivement, aux demi-noix **31** et **32.**

La noix **30** est traversée par un axe **36** sur lequel la partie distale de la tige **6** est librement enfilée pour être en appui sur la demi-noix **32.** La partie terminale de l'axe **36** coopère, par une partie filetée **37,** avec un écrou **38** ou autre moyen de blocage, prévu pour assumer une pluralité de fonctions qui ressortent de ce qui suit.

L'examen de la **fig. 4** permet de constater que l'axe **z-z'** de la tige de visée extramédullaire **6** est situé à une distance du centre **O,** telle qu'elle définit un rayon **R** qui est égal à la mesure ou à la distance de déport **d** comprise entre l'axe du fût **12** et l'axe de la broche de pointage **17.**

L'instrumentation ancillaire tibiale comprend, en outre, un support de tête réglable **40** qui est constitué par un tube **41** comportant une partie cylindrique **41a** et une partie à section polygonale **41b,** par exemple carrée, par laquelle le tube **41** est monté coulissant dans un logement traversant **42** offert par le manchon **10,** parallèlement à l'orientation angulaire obligée de la potence et perpendiculairement à l'axe **z-z'**. La position du tube **41** peut être réglée par l'intermédiaire d'un moyen d'immobilisation **43,** tel qu'une vis pointeau ou analogue.

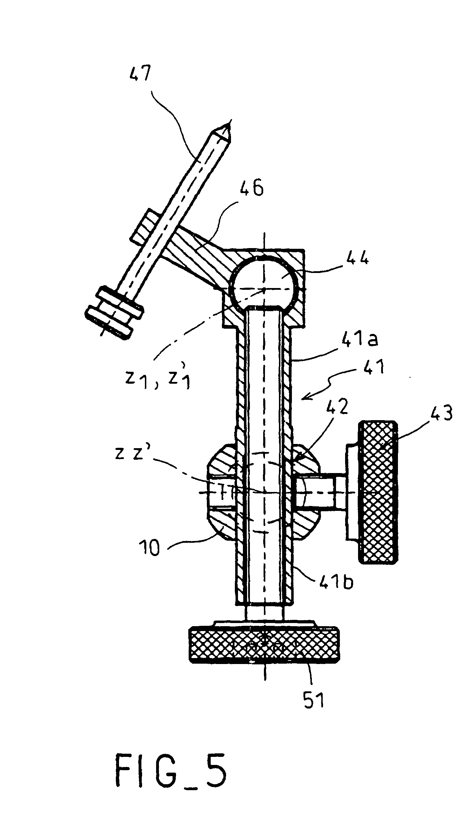

Le tube coulissant **41** porte, à son extrémité orientée dans le sens de la potence **9,** une bague **44,** de préférence tubulaire et cylindrique, dont l'axe **z**_{**1**}**-z'**_{**1**} est parallèle à l'axe **Z-Z'.** La bague **44** est prolongée latéralement par une patte **46** susceptible d'être traversée par une broche **47 (fig. 5).** La bague **44** est destinée à permettre le support d'une tête de travail **48** montée par l'intermédiaire d'une colonne graduée **50** engageable dans la bague **44.** La position relative axiale de la colonne graduée **50** est réglable par l'intermédiaire d'un organe de blocage, tel que **51,** qui est, par exemple, constitué par une vis d'immobilisation montée dans un taraudage du tube **41** pour être accessible à l'opposé de la bague **44.**

La tête de travail **48,** à caractère amovible et réglable, comprend un plateau **52** à la base duquel est formée la colonne **50** et sur la surface supérieure duquel est formée une glissière **53** définissant un plan d'appui et de référence **P-P' (fig. 1).** La glissière **53** présente une orientation générale perpendiculaire à l'axe **z-z'** et orthogonale à la direction du tube **41** et de la potence **9.** La glissière **53** est prévue pour le montage d'un guide **54** susceptible de coulisser sur la glissière et d'être réglé en position, par l'intermédiaire d'un organe d'immobilisation **53a.**

Le guide **54** comprend un corps **55** formant, dans l'exemple représenté, une première branche **56** délimitant une fente-guide de coupe **57** et une seconde branche **58** délimitant aussi une fente-guide de coupe **59.** Le corps **54** est, plus particulièrement, réservé à la résection d'un demi-plateau tibial et, dans cette optique, la fente-guide de coupe **57,** dite de première fonction, présente un plan qui est parallèle au plan de référence **P-P',** alors que la fente **59,** dite de seconde fonction, présente un plan qui lui est perpendiculaire.

Le guide **54** comporte, en outre, des moyens de support, d'immobilisation et de réglage d'un palpeur, de tels moyens, désignés dans leur ensemble par la référence **60,** comprenant un bloc **61** adaptable de façon amovible par une queue **62** dans un trou correspondant de la branche **56.** La queue **62** peut, avantageusement, être du type à baïonnette pour coopérer, par exemple, avec la fente **57,** assurant son immobilisation sur le corps **54.** Le bloc **61** est à même de supporter et de régler, par l'intermédiaire d'un bouton **63,** un palpeur **64** qui peut être constitué par une épingle, tige ou analogue, comportant une ou plusieurs parties terminales coudées **65.**

Le plan **P-P'** de la glissière **53** peut être situé dans un plan strictement orthogonal à l'axe de la colonne **50** ou présenter, par rapport à cet axe, une déclivité postérieure, tel que cela est illustré par la **fig. 1.** Une telle déclivité peut, par exemple, être fixée par construction à trois, six ou neuf degrés.

De même, le plan de la première fente-guide de coupe **57** peut être purement orthogonal à l'axe de la colonne **50** ou, au contraire, présenter, par rapport à ce dernier, une inclinaison de varus ou de valgus pouvant être comprise entre 0 et 5°.

L'instrumentation ancillaire tibiale décrite ci-dessus est mise en oeuvre de la façon suivante, lorsqu'il convient de réaliser la résection osseuse de la glène interne **4a** du plateau tibial **1,** en vue de l'adaptation d'un élément prothétique unicompartimental.

La tige de visée extramédullaire **6,** pourvue de la potence **9,** est disposée face à l'arête antérieure du tibia **2,** de manière que la broche **17** soit pointée sur le plateau tibial, en alignement avec l'axe **A-A'.**

La partie distale de la tige de visée **6,** adaptée sur la noix **30,** est alors approchée de la partie distale du tibia **2** et du péroné **3,** de telle manière que les contre-pointes **22** puissent être pointées sur les centres anatomiques des malléoles **23** et **24** selon une position stable conférée à l'étrier **20,** par pénétration corticale assurée par les vis **25** et sécurisée par l'actionnement des organes de blocage **26.**

Dans cette position, l'étrier **20** fait un angle déterminé par rapport à l'aplomb **z-z'**, un tel angle étant la traduction de la conformation anatomique devant être respectée en fonction de la conformation malléolaire.

L'écrou **38** est ensuite manoeuvré pour conférer une liberté suffisante de déplacement de la noix **30** à l'intérieur de la coulisse **27,** de manière à amener l'axe **z-z'** en alignement avec et parallèlement à l'axe **A-A'** mécanique du tibia **2.** Dans cette position, illustrée par la **fig. 2,** la potence est immobilisée par engagement de la broche **18** et l'écrou **38** est serré de manière à assumer, avec la coopération des organes complémentaires, d'une part, l'immobilisation de la noix dans une position déterminée à l'intérieur de la coulisse **27,** d'autre part, l'immobilisation de la tige **6** sur l'axe **37** et, par ailleurs, l'orientation spécifique conférée à l'étrier **20.**

Dans cette position, les parties télescopiques **7** et **8** ont adopté une extension relative adaptée qui est fixée par l'intermédiaire de l'organe de blocage ou de réglage **6a.**

Dans cet état et en raison des caractéristiques constructives de l'instrumentation ancillaire, l'axe **z-z'** est, de façon certaine, placé parallèlement à l'axe **A-A',** de même que l'axe **z**_{**1**}**-z'**_{**1**} de la bague **44** qui est amenée en butée contre la tubérosité antérieure **70** du tibia, par coulissement du tube **41,** ensuite immobilisé par l'organe de blocage **43.** L'immobilisation est assurée, de préférence, après insertion de la broche **47** dans le tibia, après quoi la potence **9** est dégagée par désolidarisation de la vis **14.**

La colonne **50** du plateau **52** est ensuite montée dans la bague **44,** puis la tête **48,** équipée du palpeur **60,** est montée sur la glissière **53.** Il est alors procédé à un triple réglage consistant à placer la fente-guide de coupe **59** selon l'orientation convenable par rapport au plan sagittal, à fixer le corps **55** dans la position convenable sur la glissière **53** et à régler verticalement la colonne **50** au moyen du palpeur appréciant, par exemple, l'excavation la plus profonde de la glène **4a,** pour en déterminer l'épaisseur osseuse devant être réséquée.

Lorsque la position recherchée est atteinte, les organes de blocage **53a** et **51** sont serrés, puis des broches transosseuses sont engagées à travers des trous **71** qui sont ménagés dans la partie inférieure de la branche **56.** Les trous **71** sont, par exemple, au nombre de trois et sont établis selon une direction perpendiculaire et sensiblement tangente et sous-jacente à la fente-guide de coupe **57,** de manière à établir une liaison solide entre le plateau tibial et le corps **55,** à même alors de constituer une référence d'appui pour les opérations ultérieures.

Dans cet état, le palpeur **60** est enlevé et la tige de visée médullaire **6** est ôtée par dégagement de l'étrier **20,** enlèvement de la broche **47** et coulissement de la bague **44** par rapport à la colonne **50** qui peut aussi être dégagée du corps **55** par coulissement de la glissière **53.**

Par l'intermédiaire d'une lame de scie, connue dans la technique et engagée dans la première fente **57,** il est alors possible de procéder à la résection de la glène concernée, selon un plan parallèle au plan de référence **P-P'**, tenant compte éventuellement du caractère déclive anatomique de l'articulation. Cette opération est complétée ensuite par la résection sensiblement verticale par l'intermédiaire de la fente-guide de coupe **59** qui permet de faire sauter la masse osseuse sciée pour mettre à nu la masse spongieuse subsistant sur laquelle l'implantation de la prothèse unicompartimentale de tibia doit être ultérieurement assurée.

Tel que cela ressort de ce qui précède, l'instrumentation ancillaire tibiale permet de disposer de références spatiales particulièrement précises, tenant compte des caractéristiques anatomiques pour identifier et matérialiser l'axe mécanique **A-A',** selon une référence antérieure **z-z',** à partir de laquelle la tête de travail **48** est réglée selon les orientations convenables pour placer les fentes-guides de coupe **57** et **59** en alignement avec les plans de résection recherchés.

Il doit être considéré que les moyens décrits ci-dessus fournissent un domaine référentiel stable et précis à partir duquel tous les réglages élémentaires peuvent être apportés individuellement sans incidence relative, afin de placer le corps **55** exactement dans les plans et orientations recherchés pour pratiquer les résections osseuses respectant les caractéristiques anatomiques de l'articulation. Les moyens ci-dessus peuvent être mis en oeuvre indifféremment pour une résection interne ou externe du plateau tibial, étant donné qu'il suffit, le cas échéant, de monter, sur la glissière **53,** le corps **55** après retournement de 180° ou, encore, un corps similaire, spécifiquement adapté. A cet égard, le tube **41** est rendu réversible par la partie **41a** qui permet une rotation sur son axe dans le logement **42,** en vue d'orienter la patte **46** à l'opposé de la position selon la **fig. 5.**

Il doit aussi être considéré que la glissière **53,** de préférence établie de façon symétrique sur le plateau **52,** de part et d'autre de l'axe de la colonne **50,** est à même de supporter un guide **54** permettant d'effectuer, en une seule opération, la résection pluricomparitmentale du plateau tibial **1.**

L'invention n'est pas limitée aux exemples décrits et représentés car diverses modifications peuvent y être apportées sans sortir de son cadre.

## Revendications

1. Instrumentation ancillaire tibiale pour la résection osseuse de tout ou partie du plateau tibial, comprenant :
• une tige télescopique (6) réglable de visée extramédullaire définissant un axe (z-z'),
• un guide de coupe (54),
• et un palpeur de réglage (64),
**caractérisée en ce qu'**elle comprend :
• une potence (9) de pointage et d'immobilisation sur le plateau tibial, adaptée de façon amovible sur la partie supérieure ou proximale de la tige de visée (6) et pourvue d'au moins une broche (17) de pointage sur le plateau tibial,
• un étrier (20) d'alignement malléolaire délimitant une coulisse d'orientation (27),
• une noix (30) de réglage et d'immobilisation liant la partie terminale inférieure ou distale de la tige de visée (6) à la coulisse (27),
• un support de tête réglable (40) porté par la partie de la tige voisine de la potence (9),
• et une tête de travail (48) amovible et réglable sur le support de tête (40) et apte à recevoir le palpeur (64) de réglage et le guide de coupe (54).

2. Instrumentation ancillaire tibiale selon la revendication 1, **caractérisée en ce que** la potence de pointage (9) comprend une seconde broche (18) parallèle à la broche de pointage (17).

3. Instrumentation ancillaire tibiale selon la revendication 1 ou 2, **caractérisée en ce que** l'étrier (20) d'alignement malléolaire comprend deux contre-pointes opposées (22) définissant un axe d'alignement malléolaire (x-x') et **en ce que** la coulisse (27) est réalisée en portion de cercle défini par un centre (O) situé sur l'axe d'alignement et par un rayon (r) plaçant l'axe (z-z') de la tige (6) à une distance du centre (O) égale à la distance de déport (d) de la potence (9) qui est orientée perpendiculairement à la tige.

4. Instrumentation ancillaire tibiale selon la revendication 3, **caractérisée en ce que** les contre-pointes (22) sont portées par deux branches (21) de l'étrier (20) prolongeant de part et d'autre la coulisse (27) et sont associées à des vis (25) de pénétration osseuse et à des organes de blocage (26).

5. Instrumentation ancillaire tibiale selon l'une des revendications 1 à 4, **caractérisée en ce que** la coulisse (27) coopère avec la noix (30) qui comprend deux demi-noix (31 et 32) associées par un axe (36) traversant librement la partie distale de la tige de visée (6) et portant un moyen (38) assurant une fonction d'immobilisation de la noix dans la coulisse et de la tige sur l'axe (36) et une fonction d'orientation du plan de l'étrier (20) par rapport à l'axe géométrique de la tige.

6. Instrumentation ancillaire tibiale selon la revendication 1, **caractérisée en ce que** le support de tête (40) comprend:
• un tube (41) porté par la partie proximale de la tige de visée (6) pour s'étendre, à distance et sous la potence de pointage (9), perpendiculairement à l'axe de la tige et dans le même sens que la potence et l'étrier,
• un moyen (43) de réglage de la position axiale du tube sur la tige,
une bague cylindrique (44) adaptée en bout du tube et dont l'axe est perpendiculaire à celui du tube et parallèle à celui de la tige,
• et un moyen (51) d'immobilisation propre à la bague (44).

7. Instrumentation ancillaire tibiale selon la revendication 6, **caractérisée en ce que** la tête de travail (48) comprend un plateau porteur (52) pourvu
• d'une colonne graduée (50) apte à être montée et réglée axialement dans la bague (44),
• d'une glissière (53) d'axe orthogonal à l'axe de la colonne et définissant un plan de référence et d'appui (**P-P'**),
• et du guide (54) déplaçable et réglable sur la glissière.

8. Instrumentation ancillaire tibiale selon la revendication 7, **caractérisée en ce que** le plan de référence (P-P') de la glissière présente une déclivité postérieure.

9. Instrumentation ancillaire tibiale selon la revendication 7, **caractérisée en ce que** le guide (54) présente des trous (71) d'engagement de broches d'immobilisation osseuse.

10. Instrumentation ancillaire tibiale selon la revendication 7 ou 9, **caractérisée en ce que** le guide (54) définit au moins un première fente-guide de coupe (57) et porte un bloc amovible (60) de support et de réglage d'un palpeur (64).

11. Instrumentation ancillaire tibiale selon la revendication 10, **caractérisée en ce que** le guide (54) définit une première fente-guide de coupe (57) déportée latéralement par rapport à l'axe de la colonne (50) pour la résection d'une partie latérale du plateau tibial et une seconde fente-guide de coupe (59) perpendiculaire à la première.

12. Instrumentation ancillaire tibiale selon l'une des revendications 9 à 11, **caractérisée en ce que** le guide (54) comporte une base d'adaptation sur la glissière et à partir de laquelle s'élève un corps (55) en forme d'équerre dont les branches définissent les fentes-guides de coupe, l'une desdites branches présentant des moyens d'adaptation du bloc amovible (60).

13. Instrumentation ancillaire tibiale selon l'une des revendications 10 à 12, **caractérisée en ce que** le plan de la première fente-guide de coupe (57) est parallèle au plan de référence (P-P') de la glissière.

## Patentansprüche

1. Tibiales Hilfsinstrument zur Knochenresektion des gesamten Tibia-Plateaus oder Teilen davon, umfassend:
- eine einstellbare Teleskopstange (6) zur extramedullären Visur, die eine Achse (z-z') bildet,
- eine Schnittführung (54),
- und einen Einstellfühler (64),
**dadurch gekennzeichnet, dass** es umfasst:
• einen Arm (9) zum Ausrichten auf das und Fixieren auf dem Tibia-Plateau, der auf dem oberen oder proximalen Teil der Visurstange (6) abnehmbar angesetzt ist und mit wenigstens einem Stift (17) zum Ausrichten auf das Tibia-Plateau versehen ist,
• einen Bügel (20) zur malleolären Ausrichtung, der eine Lagegleitführung (27) begrenzt,
• einen Zapfen (30) zum Einstellen und Fixieren, der das untere oder distale Endteil der Visurstange (6) mit der Gleitführung (27) verbindet,
• einen Träger mit verstellbarem Kopf (40), der von dem in der Nähe des Arms (9) gelegenen Teil der Stange getragen wird,
• und einen abnehmbaren Arbeitskopf (48), der auf den Trägerkopf (40) einstellbar ist und den Einstellfühler (64) und die Schnittführung (54) aufzunehmen vermag.

2. Tibiales Hilfsinstrument nach Anspruch 1,
**dadurch gekennzeichnet, dass** der Richtarm (9) einen zweiten Stift (18) umfasst, der parallel zum Richtstift (17) verläuft.

3. Tibiales Hilfsinstrument nach Anspruch 1 oder 2,
**dadurch gekennzeichnet, dass** der Bügel (20) zur malleolären Ausrichtung zwei sich gegenüberliegende Gegenhalter (22) umfasst, die eine malleoläre Ausrichtachse (x-x') bilden, und dass die Gleitführung (27) als Teil eines Kreises ausgeführt ist, der durch einen auf der Ausrichtachse angeordneten Mittelpunkt (O) und einen Radius (r) gebildet wird, der die Achse (z-z') der Stange (6) in einer Entfernung zum Mittelpunkt (0) platziert, die dem Abstand des Versatzes (d) des Arms (9) entspricht, der lotrecht zur Stange ausgerichtet ist.

4. Tibiales Hilfsinstrument nach Anspruch 3,
**dadurch gekennzeichnet, dass** die Gegenhalter (22) von zwei Armen (21) des Bügels (20) getragen werden, die die Gleitführung (27) zu beiden Seiten verlängern und Knochenschrauben und Blockierorganen (26) zugeordnet sind.

5. Tibiales Hilfsinstrument nach einem der Ansprüche 1 bis 4,
**dadurch gekennzeichnet, dass** die Gleitführung (27) mit dem Zapfen (30) zusammenwirkt, der zwei Zapfenhälften (31 und 32) aufweist, die durch eine Achse (36) verbunden sind, die das distale Teil der Visurstange (6) ungehindert durchquert und ein Mittel (36) trägt, dessen Funktion darin besteht, den Zapfen in der Gleitführung und die Stange auf der Achse (36) zu fixieren, und auch die Aufgabe hat, die Ebene des Bügels (20) in Bezug auf die geometrische Achse der Stange auszurichten.

6. Tibiales Hilfsinstrument nach Anspruch 1,
**dadurch gekennzeichnet, dass** der Kopfträger (40) umfasst:
• ein Rohr (41), das durch das proximale Teil der Visurstange (6) getragen wird, um mit Abstand zum Richtarm und unterhalb von diesem lotrecht zur Achse der Stange und in der gleichen Richtung wie der Arm und der Bügel zu verlaufen,
• ein Mittel (43) zum Einstellen der axialen Position des Rohrs auf die Stange, einen zylindrischen Ring (44), der am Ende des Rohrs aufgesteckt ist und dessen Achse lotrecht zur Achse des Rohrs und parallel zur Achse der Stange verläuft,
• und ein Fixiermittel (51), das dem Ring (44) zugehörig ist.

7. Tibiales Hilfsinstrument nach Anspruch 6,
**dadurch gekennzeichnet, dass** der Arbeitskopf (48) eine Trägerplatte (52) umfasst, die versehen ist mit:
• einer skalierten Säule (50), die axial in dem Ring (44) angebracht und eingestellt zu werden vermag,
• einer Schiene (53) mit einer Achse, die rechtwinklig zur Achse der Säule verläuft und eine Bezugs- und Auflageebene (P-P') bildet,
• und der in der Schiene verschiebbaren und einstellbaren Führung (54).l

8. Tbiales Hilfsinstrument nach Anspruch 7,
**dadurch gekennzeichnet, dass** die Bezugsebene (P-P') der Schiene eine hintere Neigung aufweist.

9. Tibiales Hilfsinstrument nach Anspruch 7,
**dadurch gekennzeichnet, dass** die Führung (54) Löcher (71) zum Einführen von Knochenfixierstiften aufweist

10. Tibiales Hilfsinstrument nach Anspruch 7 oder 9,
**dadurch gekennzeichnet, dass** die Führung (54) wenigstens einen ersten Schnittführungsspalt (57) bildet und eine abnehmbare Einheit (60) zum Halten und Einstellen eines Fühlers (64) trägt.

11. Tibiales Hilfsinstrument nach Anspruch 10,
**dadurch gekennzeichnet, dass** die Führung (54) einen ersten Schnittführungsspalt (57) bildet, der zur Resektion eines Seitenteils des Tibia-Plateaus in Bezug auf die Achse der Stütze (50) seitlich versetzt ist, sowie einen zweiten Schnittführungsspalt (59), der lotrecht zum ersten verläuft.

12. Tibiales Hilfsinstrument nach einem der Ansprüche 9 bis 11,
**dadurch gekennzeichnet, dass** die Führung (54) eine Basis zum Ansetzen an die Schiene umfasst, von der aus sich ein rechtwinkliger Körper (55) erhebt, dessen Arme die Schnittführungsspalten bilden, wobei einer der Arme Mittel zum Ansetzen der abnehmbaren Einheit (60) aufweist.

13. Tibiales Hilfsinstrument nach einem der Ansprüche 10 bis 12,
**dadurch gekennzeichnet, dass** die Ebene des ersten Schnittführungsspalts (57) parallel zur Bezugsebene (P-P') der Schiene verläuft.

## Claims

1. Ancillary tibial instrumentation for the partial or complete bone resection of the tibial plate including:
• adjustable telescopic extramedullary aimer rod (6) defining axis z-z'
• resection guide (54)
• adjustment bar (64) featuring:
• directional fixation bracket (9) on the tibial plate, attachable to the upper or proximal part of the aimer rod (6) and equipped with one or more guide pins (17) at the tibial plate
• directional slide (27) controlled by a malleolar alignment stirrup (20)
• adjustable locking clamp (30) connecting the inferior or distal end of the aimer rod (6) to the slide (27)
• adjustable edge support (40) attached to the rod near the bracket (9)
• detachable/ adjustable working head (48) on the edge support (40) configured to connect to the bar (64) and resection guide (54).

2. Ancillary tibial instrumentation as described in claim 1 and also featuring a second pin (18) on the directional bracket (9) parallel to the guide pin (17).

3. Ancillary tibial instrumentation as described in claim 1 or 2 and also featuring a malleolar alignment stirrup (20) with two opposing centrepoints (22) defining malleolar alignment axis x-x' where the slide (27) moves in a circle around centre O on the alignment axis with radius *r* placing axis z-z' of the rod (6) at a distance from centre O equal to offset distance ***d*** of the bar (9) positioned perpendicular to the rod.

4. Ancillary tibial instrumentation as described in claim 3 and also featuring centrepoints (22) attached to two arms (21) on the stirrup (20) extending the slide (27) in either direction and including bone penetration screws (25) and blocking mechanisms.

5. Ancillary tibial instrumentation as described in claims 1 through 4 and also featuring a slide (27) working with the clamp (30) including two semiclamps (31, 32) configured on one axis (36) to move freely along the distal portion of the aimer rod (6) and equipped with a mechanism (38) for locking the clamp on the slide and the rod on the axis (36) as well as directional control of the stirrup (20) in relation to the geometric axis of the rod.

6. Ancillary tibial instrumentation as described in claim 1 and also featuring an edge support (40) including:
• a tube (41) extending from the proximal part of the aimer rod (6) along under the directional bracket (9) perpendicular to the axis of the rod in the same direction as the bracket and stirrup
• a mechanism (43) for adjusting the axial position of the tube on the rod and a cylindrical ring (44) fitted at the end of the tube with axis perpendicular to that of the tube and parallel to that of the rod
• a locking mechanism (51) on the ring (44).

7. Ancillary tibial instrumentation as described in claim 6 and also featuring a working head (48) including a carrier (52) equipped with:
• graduated column (50) configured for axial mounting and adjustment in the ring (44)
• slide mechanism (53) positioned orthogonally to the column to provide support and act as reference plane **P-P'**
• movable/adjustable guide (54) on the slide mechanism.

8. Ancillary tibial instrumentation as described in claim 7 and also featuring a slide control reference plane P-P' with posterior tilt.

9. Ancillary tibial instrumentation as described in claim 7 and also featuring a guide (54) with starter holes (71) for the skeletal fixation pins.

10. Ancillary tibial instrumentation as described in claim 7 or 9 and also featuring a guide (54) with one or more resection guide slots (57) and a movable/adjustable mount (60) for the arm (64).

11. Ancillary tibial instrumentation as described in claim 10 and also featuring a guide (54) with one resection guide slot (57) offset laterally to the axis of the column (50) for lateral resection of the tibial plate and a second resection guide slot (59) perpendicular to the first.

12. Ancillary tibial instrumentation as described in claims 9 through 11 and also featuring a guide (54) with a fitted base on the slide mechanism from which extends an angled device (55) with arms defining the resection guide slots, one arm configured to fit the movable mount (60).

13. Ancillary tibial instrumentation as described in claims 10 through 12 and also featuring a main resection guide slot plane (57) configured parallel to the reference plane (P-P¹) of the slide mechanism.
